# EUROPÄISCHE PATENTANMELDUNG

(11) **EP 2 056 220 A1**
(43) Veröffentlichungstag der Anmeldung: **06.05.2009**
(21) Anmeldenummer: 07119505.1
(22) Anmeldetag: 29.10.2007
(51) Int. Cl.: G06F 17/30

(54) **Verfahren zur kontextsensitiven Bereitstellung von patientenbezogenen Informationen**

(71) Anmelder: CompuGroup Holding AG, 56070 Koblenz (DE)
(72) Erfinder: Rodorff, Werner, 64625 Bensheim (DE)
(74) Vertreter: Richardt, Markus Albert

(57) **Zusammenfassung**

Die Erfindung betrifft ein Verfahren zur kontextsensitiven Bereitstellung von patientenbezogenen Informationen auf einer graphischen Benutzeroberfläche eines Datenverarbeitungssystems (100) mit den folgenden Schritten:
- Empfang von Daten von einem Anwendungsprogramm (108), wobei das Anwendungsprogramm (108) ein erstes Anzeigefenster (132) auf der graphischen Benutzeroberfläche (200) umfasst, wobei die Daten Patientendaten enthalten,
- Zugriff auf eine erste Datenbank (122), wobei die erste Datenbank (122) medizinische Informationsobjekte enthält, wobei die medizinischen Informationsobjekte mit Abfragebedingungen bezüglich der empfangenen Daten verknüpft sind, wobei die erste Datenbank (122) in dem Datenverarbeitungssystem enthalten ist,
- Überprüfung ob zumindest eine der Abfragebedingungen erfüllt ist,
- Erzeugung eines Popups (134) auf der graphischen Benutzeroberfläche (200), wenn zumindest eine der Abfragebedingungen erfüllt ist, wobei das Popup (134) die Informationsobjekte aufweist, für welche die Abfragebedingung erfüllt sind, wobei ein Fensterfokus auf dem ersten Anzeigefenster erhalten bleibt und wobei das Popup (134) auf der graphischen Benutzeroberfläche (200) so angezeigt wird, dass eine weitere Eingabe von Daten in dem ersten Anzeigefenster nicht beeinträchtigt wird.

## Beschreibung

Die Erfindung betrifft ein Verfahren zur kontextsensitiven Bereitstellung von patientenbezogenen Informationen auf einer grafischen Benutzeroberfläche eines Datenverarbeitungssystems, ein Datenverarbeitungssystem und ein Computerprogrammprodukt. Medizinische Informationssysteme dokumentieren unter anderem vielfältige, patientenbezogene, administrative und medizinische Daten. Aufgrund des stetig wachsenden medizinischen Wissens und der Komplexität der vom Arzt erhobenen, patientenbezogenen medizinischen Daten müssen für moderne medizinische Informationssysteme durch kontextsensitive medizinische unterstützende Systeme, im Folgenden "Decision Support Systeme", bereitgestellt werden, welche medizinisches Personal bei deren täglichen Arbeit unterstützen. Kontextsensitiv bedeutet in diesem Zusammenhang, dass ein Arzt während der Dokumentation der medizinisch relevanten Daten eines Patienten aufgrund der administrativen Daten wie Alter, Geschlecht oder Diagnosen, Befunde, Therapien, Verordnungen usw., auf besondere Umstände oder Informationsangebote, die zum Kontext der aktuellen medizinischen Behandlung des Patienten passen, hingewiesen wird. So kann ein solches System beispielsweise vertiefende Literaturangebote, neueste medizinische Studienergebnisse oder sonstige Information anbieten oder auf bestimmte Umstände, wie mögliche Unverträglichkeiten von Verordnungen oder Risiken, die zur aktuellen Krankheits- oder Behandlungssituation des Patienten passen, hinweisen. Des Weiteren können allgemeine Informationen, passend zur Gesundheitssituation des Patienten, wie beispielsweise Gesundheits- oder Ernährungshinweise, gegeben werden. Dies führt somit dazu, dass zum einen der Arzt kontinuierlich während der Behandlung seiner Patienten mit fachspezifischen Hinweisen unterstützt wird. Zum anderen hat dies den Vorteil, dass eine manuelle zusätzliche Suche des Arztes in entsprechenden Datenbanken oder einschlägiger Literatur entfällt. Damit werden wertvolle Systemressourcen von medizinischen Informationssystemen durch aufwändige und allgemein gehaltene Datenbankrecherchen nicht verschwendet, was insbesondere medizinische Informationssysteme, zum Beispiel in Krankenhäusern mit einer Vielzahl von Computerterminals und Zugriffspunkten, in wesentlicher Weise entlastet.

Demgegenüber liegt die Aufgabe zugrunde, ein verbessertes Verfahren zur kontextsensitiven Bereitstellung von patientenbezogenen Informationen auf einer grafischen Benutzeroberfläche eines Datenverarbeitungssystems, ein verbessertes Datenverarbeitungssystem und ein verbessertes Computerprogrammprodukt zu schaffen.

Die der Erfindung zugrunde liegenden Aufgaben werden jeweils mit den Merkmalen der unabhängigen Patentansprüche gelöst. Bevorzugte Ausführungsformen der Erfindung sind in den abhängigen Patentansprüchen angegeben.

Erfindungsgemäß wird ein Verfahren zur kontextsensitiven Bereitstellung von patientenbezogenen Informationen auf einer grafischen Benutzeroberfläche eines Datenverarbeitungssystems geschaffen. Das Verfahren umfasst dabei den Schritt des Empfangens von Daten von einem Anwendungsprogramm, wobei das Anwendungsprogramm ein erstes Anzeigefenster auf der grafischen Benutzeroberfläche umfasst, wobei die Daten Patientendaten enthalten. In einem weiteren Schritt erfolgt der Zugriff auf eine erste Datenbank, wobei die erste Datenbank medizinische Informationsobjekte enthält, wobei die medizinischen Informationsobjekte mit Abfragebedingungen bezüglich der empfangenen Daten verknüpft sind, wobei die erste Datenbank in dem Datenverarbeitungssystem enthalten ist. In einem weiteren Schritt erfolgt schließlich die Überprüfung, ob zumindest eine der Abfragebedingungen erfüllt ist und die Erzeugung eines Popups auf der grafischen Benutzeroberfläche, wenn zumindest eine der Abfragebedingungen erfüllt ist. Dabei weist das Popup die Informationsobjekte auf, von welchen die Abfragebedingungen erfüllt sind, wobei ein Fensterfokus auf dem ersten Anzeigefenster erhalten bleibt und wobei das Popup auf der grafischen Benutzeroberfläche so angezeigt wird, dass eine weitere Eingabe von Daten in dem ersten Anzeigefenster nicht beeinträchtigt wird. Unter Patientendaten werden im Folgenden Daten über den Patienten, als auch über den behandelnden Arzt, die Arztpraxis etc. verstanden.

Das erfindungsgemäße Verfahren hat den Vorteil, dass der Arbeitsfluss eines Anwenders bei der Verwendung des Anwendungsprogramms nicht unterbrochen wird und der Anwender dennoch in gezielter Weise auf die Informationsobjekte hingewiesen wird. Außerdem hat das erfindungsgemäße Verfahren den Vorteil, dass medizinische Informationssysteme in ihrer Grundstruktur, d.h. bezüglich des Anwendungsprogramms, mit seinem ersten Anzeigefenster unverändert bleiben können, während lediglich Abfragebedingungen und die erste Datenbank regelmäßig mit neuesten Informationen aktualisiert werden. Damit ist ein Programm-Update im klassischen Sinne nicht mehr notwendig, und es genügt lediglich ein Datenbank-Update, womit der technische Aufwand sowohl für Verwender der Datenverarbeitungssysteme in Form von medizinischen Informationssystemen als auch für jene Dienstanbieter, welche Zusammenstellungen von Informationsangeboten und Informationsobjekten anbieten, wesentlich reduziert ist. Somit ist es möglich, zu beliebigen Zeitpunkten neue Verknüpfungen und Dateninhalte an die angeschlossenen Anwendungsprogramme anzubinden, ohne die Anwendungsprogramme selbst zu modifizieren - es genügt hier die einmalige Bereitstellung einer Schnittstelle der Anwendungsprogramme zum Decision Support System.

Hierbei ist insbesondere zu berücksichtigen, dass eine Minimierung des technischen Aufwandes insbesondere deshalb relevant ist, da die Anzahl der möglichen Verknüpfungskomponenten von medizinischen Informationselementen außerordentlich hoch ist und einem permanenten Wandel unterliegt, da sich das Informationsangebot stetig und immer schneller erhöht.

Nach einer Ausführungsform der Erfindung weist das Popup ferner eine Verknüpfung mit weiterführenden Informationen auf, wobei bei einer Benutzeraktivierung der Verknüpfung ein Lesen der weiterführenden Informationen von einer Informationsquelle erfolgt und eine Ausgabe der ausgelesenen weiterführenden Informationen in einem zweiten Anzeigefenster auf der grafischen Benutzeroberfläche erfolgt. Dabei handelt es sich bei der Informationsquelle um die erste Datenbank oder eine dem Datenverarbeitungssystem externe Datenquelle, wie zum Beispiel das Internet und/oder eine weitere zweite Datenbank.

Dies bedeutet, dass ein Anwender frei in der Entscheidung ist, durch Benutzeraktivierung der Verknüpfung das Informationsangebot, welches im Popup erscheint, anzunehmen und damit auf Wunsch weitergehende Informationen abzurufen. Der Vorteil der Verwendung einer externen Datenquelle, wie zum Beispiel das Internet und/oder eine weitere Datenbank, ist dabei, dass tagesaktuelle Informationen in dem zweiten Anzeigefenster auf der grafischen Benutzeroberfläche bereitgestellt werden können. So ist es zum Beispiel möglich, die erste Datenbank lediglich vierteljährlich zu aktualisieren, womit der Aufwand für einen Benutzer des Datenverarbeitungssystems in zeitlicher und technischer Hinsicht reduziert wird. Hingegen ist durch die Verwendung der externen Datenquelle dennoch gewährleistet, dass zum Beispiel aktuelle Preisinformationen zu Medikamenten, aktuelle klinische Fallstudien, aktuelle Empfehlungen zum Beispiel bezüglich Impfungen bei Reisen in bestimmten Ländern usw., zur Verfügung gestellt werden können.

Nach einer Ausführungsform der Erfindung wird das Popup von der grafischen Benutzeroberfläche nach einem vorgegebenen ersten Zeitintervall entfernt.

Nach einer weiteren Ausführungsform der Erfindung sind die weiterführenden Informationen in der Datenbank als HTML- oder XML-Dokumente gespeichert. Dabei handelt es sich bei dem zweiten Anzeigefenster vorzugsweise um ein Fenster eines Webbrowsers. Die Verwendung eines Fensters eines Webbrowsers als zweites Anzeigefenster hat dabei den Vorteil, dass das erfindungsgemäße Verfahren zur kontextsensitiven Bereitstellung von patientenbezogenen Informationen auf nahezu jedem beliebigen Datenverarbeitungssystem ergänzend implementiert werden kann, da Datenverarbeitungssysteme heutzutage standardmäßig über Webbrowser verfügen. Somit muss das Anwendungsprogramm lediglich ein Interface zur Verfügung stellen, über welches ein im Hintergrund laufendes Decision Support System die Daten von dem Anwendungsprogramm empfängt, um daraufhin unter Verwendung des Webbrowsers die Popups bzw. weiterführenden Informationen zur Verfügung zu stellen.

Nach einer Ausführungsform der Erfindung enthalten die von dem ersten Anzeigefenster empfangenen Daten Informationen über den Benutzer der grafischen Benutzeroberfläche. Dabei umfassen die Informationen über den Benutzer Praxisstammdaten und/oder eine Facharztgruppe und/oder eine Benutzerkennung.

Nach einer weiteren Ausführungsform der Erfindung umfasst das Verfahren ferner den Schritt des Abfragens ergänzender, zu dem Patienten zugehöriger Patientendaten von einer dritten Datenbank, wobei die dritte Datenbank Teil des Datenverarbeitungssystems oder eine externe Datenbank ist, wobei die medizinischen Informationsobjekte zusätzlich mit Abfragebedingungen bezüglich der abgefragten ergänzenden Patientendaten verknüpft sind.

Die Verwendung einer dritten Datenbank zum automatischen Abfragen ergänzender, zu dem Patienten zugehöriger Patientendaten hat dabei den Vorteil, dass in die Entscheidungsfindung bezüglich des Anzeigeinhalts der Popups auch Informationen einfließen können, welche nicht nur ausschließlich von dem Datenverarbeitungssystem stammen, auf welchem das Anwendungsprogramm aktuell läuft. Beispielsweise ist das Szenario denkbar, dass ein Patient zuvor in einer Arztpraxis war und daraufhin in eine Klinik überwiesen wurde, welche das Verfahren zur kontextsensitiven Bereitstellung von patientenbezogenen Informationen verwendet. In diesem Fall werden zum Beispiel bei der Eingabe von Patientendaten durch einen Arzt im Hintergrund von der überweisenden Arztpraxis die besagten, ergänzenden, zu dem Patienten zugehörigen Patientendaten abgefragt, um somit ein vollständiges Bild über den Gesundheitszustand, die bisherige Medikation, Anamnesedaten, Therapien, usw. zu erhalten.

Nach einer Ausführungsform der Erfindung umfassen die Abfragebedingungen personalisierte Benutzerabfragebedingungen und/oder personalisierte Patientenabfragebedingungen, wobei die Benutzerabfragebedingungen und Patientenabfragebedingungen dynamisch in der ersten Datenbank ergänzt werden. Dies bedeutet, dass zum Beispiel Abfragebedingungen verwendet werden, welche in ganz spezieller Weise auf einen behandelnden Arzt, zum Beispiel bezüglich dessen Arbeitsgebiet (Facharztgruppe), zugeschnitten sind.

Nach einer Ausführungsform der Erfindung weist das Popup ferner zumindest eine durch den Benutzer auswählbare Anzeigeoption aus, wobei bei einer Auswahl der Anzeigeoption die personalisierte Benutzerabfragebedingung und/oder Patientenabfragebedingung erzeugt wird, wobei in die personalisierte Benutzerabfragebedingung und/oder Patientenabfragebedingung die durch den Benutzer ausgewählte Anfrageoption eingeht. In einem praktischen Beispiel handelt es sich dabei bei der Anzeigeoption um das sofortige Ausblenden und Wiedereinblenden des Popups nach einem vorgegebenen auswählbaren zweiten Zeitintervall und/oder es handelt sich um das sofortige Ausblenden des Popups und nicht Wiedereinblenden des Popups. So kann zum Beispiel in die personalisierte Benutzerabfragebedingung eingehen, dass eine mittels des Popups eingeblendete Information nach zum Beispiel dreimaligem Auswählen der Anzeigeoption "sofortiges Ausblenden und nicht Wiedereinblenden" auch für zukünftige Dateneingaben durch diesen speziellen Arzt nicht mehr verwendet werden, da in diesem Fall davon auszugehen ist, dass der behandelnde Arzt an einer solchen Information in keiner Weise interessiert ist. Dasselbe kann in ähnlicher Weise auch spezifisch für einen bestimmten Patienten durchgeführt werden, wobei hier eine Patientenabfragebedingung dynamisch aktualisiert wird, wenn der behandelnde Arzt auswählt, dass für diesen Patienten ein bestimmter Vorschlag, zum Beispiel zur Medikamentierung oder Therapierung, ungeeignet ist.

Nach einer weiteren Ausführungsform der Erfindung weisen die empfangenen Daten Datenobjekte auf, wobei beim Empfang der Daten die Datenobjekte in einem ersten Stack gespeichert werden. Daraufhin werden aus einer ersten Tabelle für die in dem ersten Stack gespeicherten Datenobjekte diesen Datenobjekten in der ersten Tabelle zugeordnete Kennungen ausgelesen, wobei jeder Kennung eines der medizinischen Informationsobjekte zugewiesen ist. In einem weiteren Schritt wird in einem zweiten Stack zu jeder Kennung die Anzahl der in dem ersten Stack gespeicherten Datenobjekte gespeichert, welche in der ersten Tabelle dieser Kennung zugeordnet sind. Schließlich gilt die Abfragebedingung dann als erfüllt, wenn die zu einer Kennung in dem zweiten Stack gespeicherte Anzahl an empfangenen Datenobjekten der Anzahl von Datenobjekten entspricht, welche in der ersten Tabelle zu der Kennung zugeordnet sind.

Nach einer Ausführungsform der Erfindung erfolgt die Speicherung eines empfangenen Datenobjekts in dem ersten Stack nur dann, wenn das Datenobjekt noch nicht in dem ersten Stack gespeichert ist.

Die Zuordnung der Kennungen zu Datenobjekten in der ersten Tabelle in Kombination mit der beschriebenen Verwendung des ersten und zweiten Stacks, sowie der zweiten Tabelle hat dabei den Vorteil, dass eine große Anzahl an Abfragebedingungen in kürzester Zeit überprüft werden kann: Anstatt eine komplette Datenbank abzufragen, in welcher verschiedene Verknüpfungen und Bedingungen zusammen mit entsprechenden Informationsobjekten abgelegt sind, muss lediglich auf eine einzelne Tabelle und die beiden Stacks zugegriffen werden, um empfangene Datenobjekte bezüglich der Abfragebedingungen zu überprüfen. Dies ermöglicht eine Evaluierung der Abfragebedingungen in Echtzeit, selbst bei einer außerordentlich hohen Anzahl an Verknüpfungsmöglichkeiten von Datenobjekten und Kennungen, wie dies für medizinische Informationsangebote der Fall ist. Die Verwendung einer einfachen Datenbankabfrage würde enorme Systemressourcen beanspruchen, was durch die erfindungsgemäße Verwendung der Tabellen und Stacks vermieden werden kann.

In einem weiteren Aspekt betrifft die Erfindung ein Datenverarbeitungssystem zur kontextsensitiven Bereitstellung von patientenbezogenen Informationen auf einer grafischen Benutzeroberfläche des Datenverarbeitungssystems. In einem noch weiteren Aspekt betrifft die Erfindung ein Computerprogrammprodukt mit von einem Prozessor ausführbaren Instruktionen zur Durchführung des erfindungsgemäßen Verfahrens zur kontextsensitiven Bereitstellung von patientenbezogenen Informationen auf einer grafischen Benutzeroberfläche eines Datenverarbeitungssystems.

Im Folgenden werden Ausführungsformen der Erfindung anhand der Zeichnungen näher erläutert. Es zeigen:
- Figur 1:: ein Blockdiagramm eines erfindungsgemäßen Datenverarbeitungssystems,
- Figur 2:: eine schematische Ansicht einer Benutzeroberfläche mit Popup,
- Figur 3:: eine schematische Ansicht einer Benutzeroberfläche mit einem zweiten Anzeigefenster mit weiterführenden Informationen,
- Figur 4:: eine Übersicht der erfindungsgemäß verwendeten Tabellen und Stacks,
- Figur 5:: ein Flussdiagramm des erfindungsgemäßen Verfahrens zur kontextsensitiven Bereitstellung von patientenbezogenen Informationen.

Im Folgenden sind einander ähnliche Elemente mit denselben Bezugszeichen gekennzeichnet.

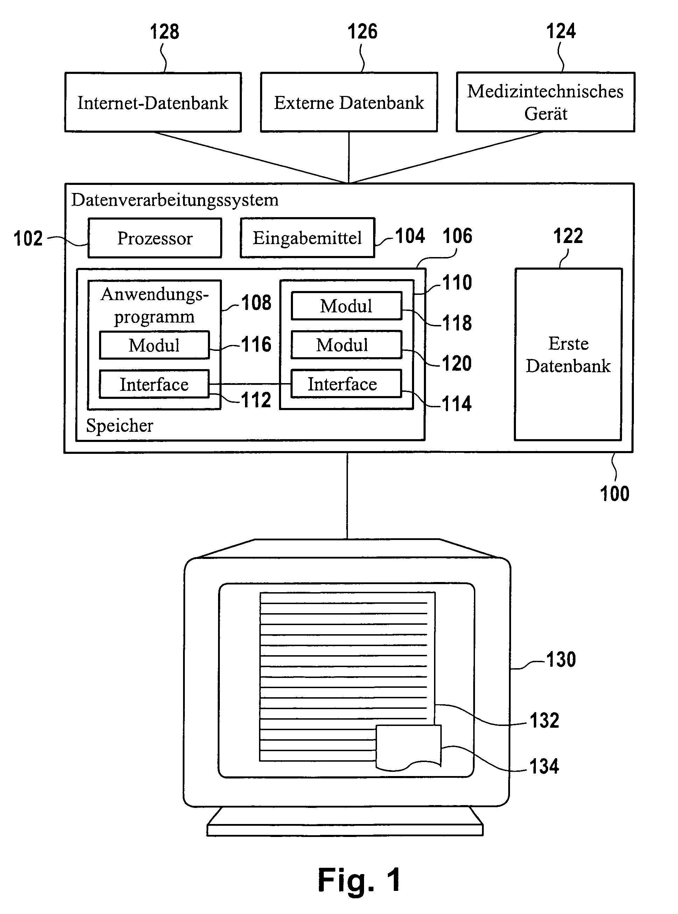

Die Figur 1 zeigt ein Blockdiagramm eines erfindungsgemäßen Datenverarbeitungssystems 100. Das Datenverarbeitungssystem 100 weist einen Prozessor 102 und Eingabemittel 104, wie zum Beispiel eine Maus, Tastatur usw., auf. Ferner weist das Datenverarbeitungssystem 100 einen Speicher 106 auf, in welchem sich ein computerausführbarer Code für ein Anwendungsprogramm 108 und ein Decision Support System 110 befindet. Des Weiteren umfasst das Datenverarbeitungssystem 100 eine erste Datenbank 122, wobei die erste Datenbank 122 medizinische Informationsobjekte enthält, welche mit Abfragebedingungen bezüglich von Daten verknüpft sind, welche von dem ersten Anwendungsprogramm 108 über dessen Interface 112 empfangen werden.

Des Weiteren angeschlossen an das Datenverarbeitungssystem 100 sind eine Internet-Datenbank 128, eine externe Datenbank 126 und beispielsweise ein medizintechnisches Gerät 124. Außerdem weist das Datenverarbeitungssystem 100 eine Anzeigevorrichtung 130 in Form eines Bildschirms auf, auf welchem, wie in der Figur 1 gezeigt, ein erstes Anzeigefenster 132 dargestellt ist, in welches ein behandelnder Arzt Patientendaten eingibt.

Während der Eingabe der Patientendaten werden nun diese Patientendaten mittels des Interface 112 vom Anwendungsprogramm 108 zum Interface 114 des Decision Support Systems 110 weitergereicht. Unter Verwendung der Programmmodule 118 und 120, welche in der Figur 1 lediglich schematisch dargestellt sind, überprüft das Decision Support System 110, ob Abfragebedingungen bezüglich der empfangenen Daten erfüllt sind. Ist dies der Fall, so wird ein Popup 134 auf der grafischen Benutzeroberfläche des Anzeigegerätes 130 erzeugt, wobei das Popup Informationsobjekte aufweist, für welche die Abfragebedingungen erfüllt sind.

Das Decision Support System 110 kann außer den Daten, welche von dem ersten Anzeigefenster 132 empfangen wurden, unter Verwendung seiner Module 118 bzw. 120 auch weiterführende Daten, zum Beispiel von der externen Datenbank 126 abfragen, wobei diese externe Datenbank zum Beispiel die Datenbank einer externen Arztpraxis, eines Pflegedienstes oder Ähnliches ist. In diesem Fall werden in einer Ausführungsform ergänzende, zu dem Patienten zugehörige Patientendaten von dieser externen Datenbank 126 abgerufen. Möglich ist es außerdem auch noch, insbesondere in Krankenhäusern, in welchen es sich bei dem Anwendungsprogramm 108 um ein Krankenhaus-Informationssystem handelt, zusätzliche medizinische Daten bezüglich des Patienten direkt von einem medizintechnischen Gerät 124, wie zum Beispiel einem Röntgengerät, einem Kernspintomografen usw. abzurufen. In diesem Fall kann das Popup 134 beispielsweise auch dazu dienen, den Arzt auf eine vorhandene Röntgenaufnahme aufmerksam zu machen, wobei in dem Popup dem Arzt eine Verknüpfung zu den dem Patienten zugehörigen Röntgenaufnahmen vorhanden ist. In diesem Fall findet eine Übertragung von z.B. Röntgen-Bilddaten lediglich dann statt, wenn ein behandelnder Arzt dies explizit wünscht, nachdem er auf das Vorhandensein solcher Bilddaten aufmerksam gemacht wurde. Dies ist insbesondere bei Krankenhaus-Informationssystemen von hoher Relevanz, da somit ein übermäßig hohes Datenaufkommen durch Datenübertragungen auf verschiedene Client-Computer des Datenverarbeitungssystems 100 vermieden wird. Damit wird die Gesamtsystemauslastung eines solchen Krankenhaus-Informationssystems erheblich reduziert.

Die Internet-Datenbank 128 dient beispielsweise dazu, um einem Benutzer in dem Popup 134 einen Link zur Verfügung zu stellen, welcher nach Aktivierung einen Benutzer zu einer entsprechenden Internetseite mit weiterführenden Informationen führt.

Wichtig in der Figur 1 ist die konsequente Trennung von Primärsystemen in Form des Anwendungsprogramms 108, zum Beispiel eines Arztinformationssystems, Krankenhaus-Informationssystems, Zahnarzt-Informationssystems usw. und dem Decision Support System 110, sodass beide aus Sicht des zugrunde liegenden Betriebssystems getrennt voneinander operierende Softwareprogramme sind. Das Anwendungsprogramm 108 stellt über seine Schnittstelle 112, die idealerweise nach einmaliger Definition und Implementierung nicht mehr geändert werden muss, dem Decision Support System 110 alle anfallenden administrativen und medizinischen Daten im Behandlungsverlauf ohne weitere Prüfung zur Verfügung. Die Datenbank 122, welche nun in das Datenverarbeitungssystem 100 integriert ist, weist verschiedene Verknüpfungen von medizinischen Informationsobjekten auf. Beispielsweise "bei Alter des Patienten größer 50, Geschlecht männlich und Diagnose I50.1, Hinweis auf neue Studie zu ...". Tritt eine oder mehrerer solcher Kombinationen auf, wird im Decision Support System 110 ein Treffer ausgelöst. In jedem Treffer sind Anzeige- und Entscheidungsobjekte verknüpft, die vom Decision Support System nun ohne Unterbrechung des Workflows des Anwenders angezeigt werden, in Form eines Popups 134, auch als so genannter "Bubble" bezeichnet.

Werden die Informationsangebote, welche in Form des Popups 134 dem Benutzer präsentiert werden, vom behandelnden Arzt nicht genutzt, verschwinden diese Angebote bzw. das Popup nach einer konfigurierbaren Zeitspanne von selbst.

Da sowohl Verknüpfungen der medizinischen Datenobjekte, die beim Auftreten der entsprechenden Kombination auszulösenden Treffer und die Informationsangebote inklusive der damit verknüpften Informationen selbst, in der Datenbank 122 bzw. alternativ zum Beispiel in der Internet-Datenbank 128 liegen können, können diese kontextsensitiven Verknüpfungen und Informationen inklusive Informationsangebote durch einen einfachen Austausch der entsprechenden Datenbanken 122 bzw. Aktualisierungen im Internet aktualisiert, modifiziert und ergänzt werden. Weder am Primärsystem in Form des Anwendungsprogrammes 108 noch am Decision Support System in Form des Anwendungsprogramms 110 müssen Änderungen vorgenommen werden.

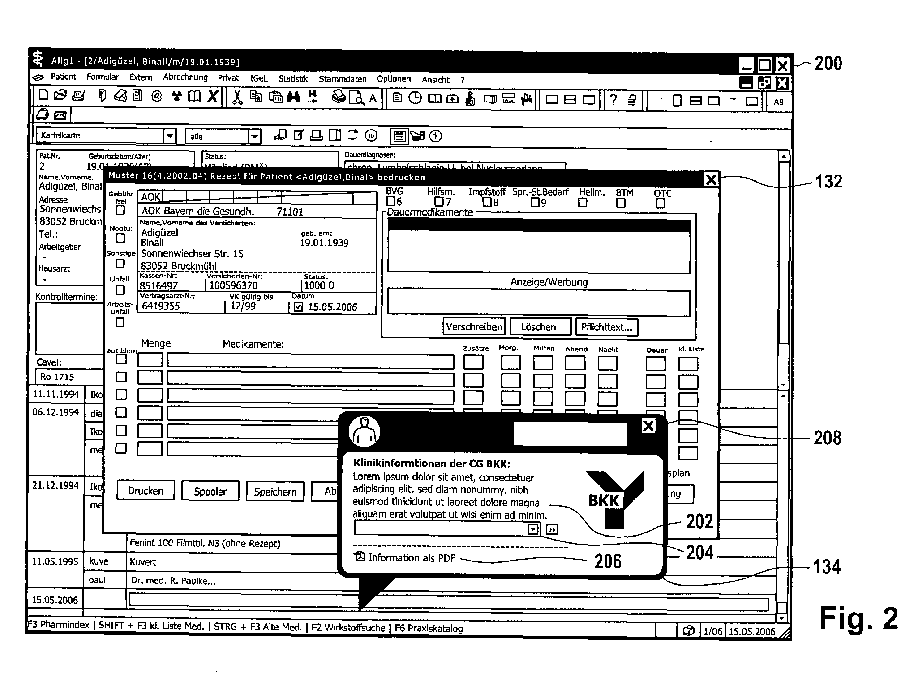

Die Figur 2 zeigt eine schematische Ansicht einer Benutzeroberfläche 200 mit Popup 134. Die grafische Benutzeroberfläche 200 weist dabei ein erstes Anzeigefenster 132 auf, welches der Arzt augenblicklich dazu verwendet, um für einen Patienten ein entsprechendes Rezept auszustellen. Während der Eingabe der Rezeptdaten erscheint nun auf der grafischen Benutzeroberfläche 200 das Popup 134, wobei das Popup auf der grafischen Benutzeroberfläche so angezeigt wird, dass eine weitere Eingabe von Daten in dem ersten Anzeigefenster möglichst nicht beeinträchtigt wird. Außerdem bleibt der Fensterfokus auf dem ersten Anzeigefenster 132 enthalten.

Das Popup 134 weist ein Informationsobjekt 202 auf, zum Beispiel weiterführende Informationen zu den im Rezept verordneten Medikamenten in Form von Nebenwirkungen. Bei Anzeige des Popups 134 hat der behandelnde Arzt nun verschiedene Möglichkeiten. Die einfachste Möglichkeit ist es, die Schaltfläche 208 zu betätigen, sodass sofort das Popup 134 von der grafischen Benutzeroberfläche 200 verschwindet. Alternativ bietet es sich an, dass der Arzt einen der Auswahlpunkte verwendet, welche ihm mittels des Drop-Down Menüs 204 zur Verfügung gestellt werden. Beispielsweise kann es sich bei einer solchen Auswahlmöglichkeit um die Möglichkeit handeln, lediglich speziell für diesen Patienten die angezeigte Information in Zukunft nicht mehr anzuzeigen. Dies würde einer so genannten personalisierten Patientenabfragebedingung entsprechen, die nach Auswahl eines solchen Punktes im Drop-Down Menü dynamisch in der ersten Datenbank 122, wie in der Figur 1 gezeigt, ergänzt wird. Ein weiterer Menüpunkt des Drop-Down Menüs 204 könnte sein, dass der Arzt in Zukunft die Anzeige weiterführender Medikamenteninformationen lediglich bezüglich eines bestimmten Herstellers nicht mehr wünscht. In diesem Fall würde eine Auswahl eines solchen Drop-Down Menüpunktes einer personalisierten Benutzerabfragebedingung genügen, welche ebenfalls dynamisch in der ersten Datenbank 122, wie in der Figur 1 gezeigt, ergänzt wird. Darüber hinaus bietet sich eine Vielzahl von Möglichkeiten, welche Menüpunkte das Drop-Down Menü 204 enthalten könnte. Dies umfasst unter anderem auch, dass beispielsweise das Popup-Fenster 134 ausgeblendet wird und nach verschieden auswählbaren Zeitspannen, beispielsweise nach 5 Minuten, 10 Minuten, oder 20 Minuten nach Schließen des ersten Anzeigefensters 132 erneut auf der grafischen Benutzeroberfläche 200 erscheint.

Ferner weist das Popup 134 eine Verknüpfung 206 auf, wobei bei Auswahl der Verknüpfung 206 ein zweites Anzeigefenster auf der grafischen Benutzeroberfläche 200 erscheint, welches weiterführende Informationen zu den Informationsobjekten 202 anzeigt.

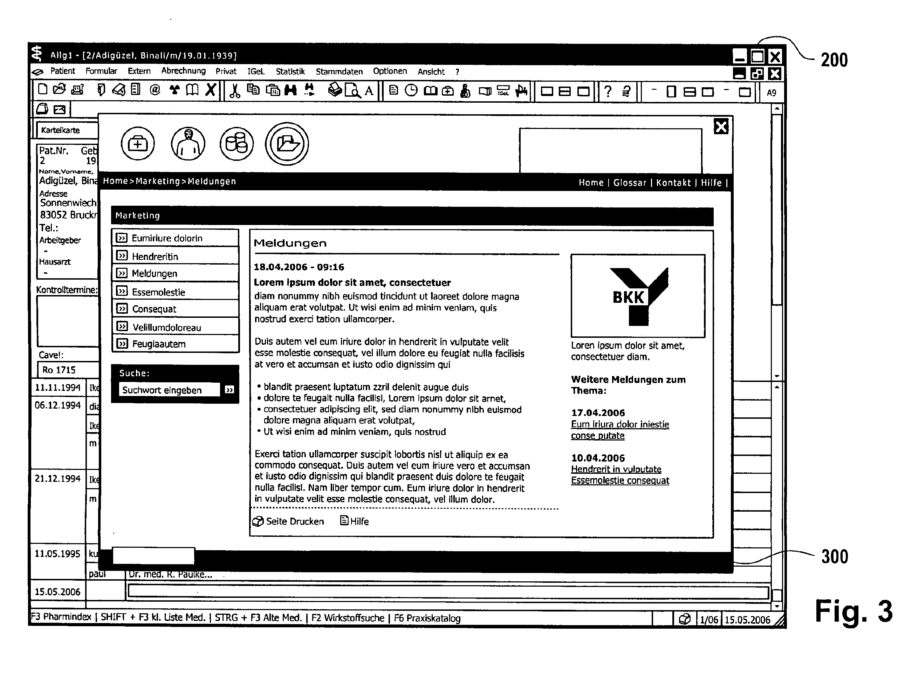

Die Figur 3 zeigt eine schematische Ansicht einer Benutzeroberfläche 200 mit einem zweiten Anzeigefenster 300 mit weiterführenden Informationen. Dieses zweite Anzeigefenster 300 ist dabei vorzugsweise ein Browserfenster, sodass hiermit unmittelbar HTML-Dateien dargestellt werden können, wie sie zum Beispiel aus entsprechenden Internet-Datenbanken heruntergeladen werden können.

In einer Ausführungsform der Erfindung bietet es sich auch an, die Popups in Verbindung mit Internet-Webservices so zu verknüpfen, dass das Popup die Verwendung eines bestimmten Webservices vorschlägt, welcher nach Bestätigung einer entsprechenden Schaltfläche im Popup in dem zweiten Anzeigefenster aufgerufen wird. Beispielsweise kann es sich bei einem solchen Webservice um einen Dienst handeln, welcher unter Verwendung der in dem ersten Anzeigefenster eingegebenen Patientenstammdaten wie Name, Vorname, Adresse, Alter, Versichertendaten usw., sowie den vom Arzt ebenfalls in dem ersten Anzeigefenster 132 verordneten Medikamenten einen Brief erstellt, welcher eine entsprechend ausformulierte persönliche Anrede und Anleitung zur Medikamenteneinnahme enthält. In diesem Fall entfällt für den Arzt die Notwendigkeit, selbst einen solchen anleitenden Brief zu verfassen, was zum einen eine Arbeitsentlastung des Arztes bedeutet, als auch zu einer Systementlastung des Datenverarbeitungssystems führt, auf welchem das Arztinformationssystem und das Decision Support System betrieben werden. Ein weiteres Beispiel für die von Verwendung von Webservices ist zum Beispiel die Möglichkeit, bei einer Überweisung eines Patienten in ein Krankenhaus dem Arzt die Möglichkeit zu geben, durch die Verwendung des Webservices für den Patienten eine geeignete Wegbeschreibung zu diesem Krankenhaus inklusive Anfahrtskizze usw. zur Verfügung zu stellen. Solche Daten sind üblicherweise auf keinem Arztrechner aufgrund der Komplexität und des Umfangs solcher Kartendaten verfügbar, sodass auch hier die Verwendung eines Webservices eine entsprechende Implementierung überhaupt erst ermöglicht.

Ein weiteres Beispiel für die Verwendung von Webservices ist, dass in Abhängigkeit von der Einweisungsdiagnose für die stationäre Behandlung und des Wohnortes des Patienten (z.B. der Postleitzahl in den Patientenstammdaten) ein für die notwendige Behandlung geeignetes oder spezialisiertes Krankenhaus in der Nähe gesucht wird. Diese Information wird daraufhin vom Webservice dem behandelnden Arzt übermittelt.

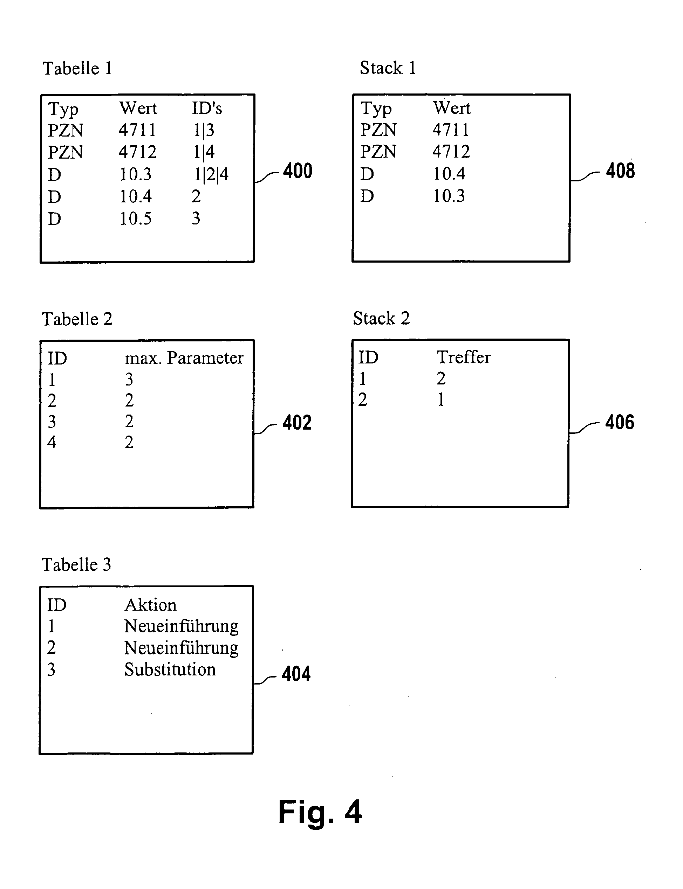

Die Figur 4 zeigt eine Übersicht der erfindungsgemäß verwendeten Tabellen und Stacks. So sind in der ersten Tabelle 400 drei Spalten vorhanden, wobei die erste Spalte den Typ des Datenobjektes angibt, wie zum Beispiel eine Pharmazentralnummer (PZN) oder einen Diagnoseschlüssel (D). Jedem eingetragenen Datenobjekt ist dabei in der Spalte "Wert" ein entsprechender Wert zugeordnet. Ferner ist jedem Datenobjekt, bestehend aus Typ und Wert, eine Kennung (ID) zugeordnet. Wie in der ersten Tabelle 400 gezeigt, können dabei mehrere verschiedene Kennungen einem Datenobjekt zugeordnet sein. Beispielsweise ist der Pharmazentralnummer PZN mit dem Wert 4711 sowohl die Kennung 1 als auch die Kennung 3 zugeordnet. Dem Diagnoseschlüssel 10.3 sind drei verschiedene Kennungen, nämlich die Kennungen 1, 2 und 4 zugeordnet.

In der zweiten Tabelle 402 ist für jede Kennung (ID) die Anzahl der Datenobjekte festgehalten, für welche die entsprechende Kennung jeweils in der ersten Tabelle 400 verschiedenen Datenobjekten zugeordnet ist. Beispielsweise ist in der ersten Tabelle 400 die Kennung 1 drei verschiedenen Datenobjekten zugeordnet, nämlich der PZN 4711, PZN 4711 und D 10.3. PZN steht hierbei für die Pharmazentralnummer eines Medikaments und D für eine Diagnose. Damit erhält in der zweiten Tabelle 402 die Kennung 1 den Wert 3. In gleicher Weise berechnet sich für die beispielhaften Kennungen 2, 3 und 4 die jeweilige Anzahl an Datenobjekten aus der ersten Tabelle 400, welche in allen Fällen für die ID = 2, 3, 4 den Wert 2 beträgt.

Die dritte Tabelle 404 weist nun für jede Kennung eine entsprechende Aktion auf, welche einem Benutzer in Form eines erscheinenden Popups als Informationsobjekt präsentiert werden soll.

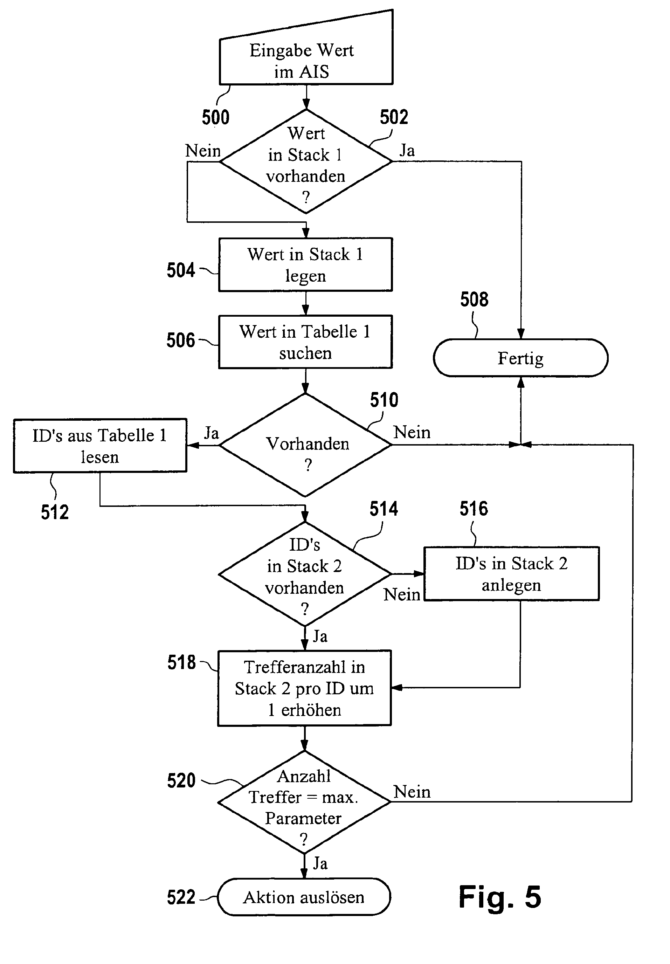

Die Verwendung der in Figur 4 gezeigten ersten und zweiten Stacks 408 bzw. 406 soll im Folgenden im Zusammenhang mit dem Flussdiagramm in Figur 5 erklärt werden, welches ein Flussdiagramm des erfindungsgemäßen Verfahrens zur kontextsensitiven Bereitstellung von patientenbezogenen Informationen darstellt. Es sei jedoch darauf hingewiesen, dass es sich hierbei lediglich um eine Ausführungsform handelt, welche auch durch andere geeignete Abfragemechanismen ersetzt werden kann, um die Erfüllung von Abfragemechanismen zu überprüfen.

So erfolgt in Schritt 500 die Eingabe eines Datenobjektes in dem ersten Anzeigefenster des Anwendungsprogramms, zum Beispiel eines Arztinformationssystems. Daraufhin wird in Schritt 502 geprüft, ob dieses Datenobjekt bereits in dem ersten Stack 408 vorhanden ist. Ist das Datenobjekt noch nicht vorhanden, so wird in Schritt 504 ein entsprechender Eintrag im ersten Stack 408 vorgenommen und in Schritt 506 daraufhin in der ersten Tabelle überprüft, ob dort das Datenobjekt einer Kennung zugeordnet ist. Ergibt sich in Schritt 510, dass das Datenobjekt in der Tat bereits einer oder mehreren Kennungen (IDs) zugeordnet ist, so werden die entsprechenden IDs in Schritt 512 aus der ersten Tabelle 400 ausgelesen.

Nun wird in Schritt 514 überprüft, ob im zweiten Stack 406 bereits Eintragungen zu diesen Kennungen existieren. Ist dies nicht der Fall, so werden die in Schritt 512 aus der ersten Tabelle ausgelesenen Kennungen nacheinander in dem zweiten Stack 406 abgelegt und außerdem für jede Kennung die Anzahl der Datenobjekte hinzugefügt, welche in dem ersten Stack 408 gespeichert sind und welche in der ersten Tabelle 400 dieser Kennung zugeordnet sind. Diese Bestimmung der Anzahl der in dem ersten Stack gespeicherten Datenobjekte, welche in der ersten Tabelle dieser Kennung zugeordnet sind, entspricht dabei dem Schritt 518 in der Figur 5. In Schritt 516 wird in dem zweiten Stack 406 lediglich die entsprechende Kennung abgelegt, und in Schritt 518 die entsprechende Trefferzahl für die Kennung um 1 erhöht. In dem praktischen Beispiel der Figur 4 ist es beispielsweise so, dass in Schritt 514 bezüglich der Kennung ID = 1 festgestellt wird, dass diese ID bereits im zweiten Stack 406 vorhanden ist. Die Kennung ID = 1 hat dabei bereits zwei Treffer, welche einer zuvor erfolgten Eingabe der Datenobjekte PZN = 4711 und PZN = 4712 (vgl. erster Stack 408) entsprechen.

Da nun zuvor in den Schritten 500 bis 512 zusätzlich das Datenobjekt D = 10,3 eingegeben wurde, wird in Schritt 514 bestimmt, dass die ID = 1 mit der Trefferanzahl = 2 in dem zweiten Stack 406 existiert. Da sowohl das Datenobjekt D = 10,3 in dem ersten Stack 408 als auch in der ersten Tabelle 400 vorhanden ist, wird nun in Schritt 518 die Trefferzahl für die Kennung ID = 1 um 1 erhöht. Daraufhin wird nun in Schritt 520 überprüft, ob die Anzahl der Treffer, nun für die Kennung ID = 1 mit Treffer = 3, der maximalen Anzahl an Datenobjekten entspricht, welche in der ersten Tabelle 400 zu dieser Kennung ID = 1 zugeordnet sind. Ein Zählvorgang der Anzahl an Datenobjekten, welche in der ersten Tabelle ID = 1 entsprechen, ist überflüssig, da aus der zweiten Tabelle 402 direkt dieser Wert (max. Parameter) ausgelesen werden kann. Ergibt sich nun, wie im vorliegenden Beispiel erklärt, dass für die Kennung ID = 1 sowohl die Trefferzahl = 3 auch der in der zweiten Tabelle 402 der Kennung ID = 1 mit max. Parameter = 3 entspricht, so wird daraufhin in Schritt 522 eine entsprechende Aktion in Form der Erzeugung eines Popups ausgelöst. Resultiert in Schritt 520, dass die Anzahl nicht dem Wert max. Parameter entspricht, so endet das Verfahren in Schritt 508.

Eine Beendigung des Verfahrens endet ebenfalls in Schritt 508, wenn zuvor in Schritt 502 bestimmt wurde, dass ein entsprechender, im Arztinformationssystem im Schritt 500 eingegebener Wert, bereits im ersten Stack 408 vorhanden ist. Der Grund ist hierbei, dass eine doppelte Zählung von bereits in das Arztinformationssystem eingegebenen Datenobjekten so verhindert werden soll. Ebenfalls endet das Verfahren in Schritt 508, wenn in Schritt 510 bestimmt wird, dass ein entsprechendes Datenobjekt, welches in Schritt 500 in das Arztinformationssystem eingegeben wurde, in der ersten Tabelle 400 nicht existiert und damit auch keiner Kennung zugeordnet ist.

Es sei noch darauf hingewiesen, dass die zweite Tabelle 402 auch vollkommen unabhängig bezüglich der maximalen Anzahl an Datenobjekten, welche eine gemeinsame Kennung ID aufweisen müssen, von der ersten Tabelle 400 erzeugt werden kann.

### Bezugszeichenliste

- 100: Datenverarbeitungssystem
- 102: Prozessor
- 104: Eingabemittel
- 106: Speicher
- 108: Anwendungsprogramm
- 110: Decision Support System
- 112: Interface
- 114: Interface
- 116: Modul
- 118: Modul
- 120: Modul
- 122: Erste Datenbank
- 124: Medizintechnisches Gerät
- 126: Externe Datenbank
- 128: Internet-Datenbank
- 130: Anzeigevorrichtung
- 132: erstes Anzeigefenster
- 134: Popup
- 200: Grafische Benutzeroberfläche
- 202: Informationsobjekt
- 204: Drop-Down Menü
- 206: Verknüpfung
- 208: Schaltfläche
- 300: zweites Anzeigefenster
- 400: erste Tabelle
- 402: zweite Tabelle
- 404: dritte Tabelle

- 406: erster Stack
- 408: zweiter Stack

## Patentansprüche

1. Verfahren zur kontextsensitiven Bereitstellung von patientenbezogenen Informationen auf einer graphischen Benutzeroberfläche eines Datenverarbeitungssystems (100) mit den folgenden Schritten:
- Empfang von Daten von einem Anwendungsprogramm (108), wobei das Anwendungsprogramm (108) ein erstes Anzeigefenster (132) auf der graphischen Benutzeroberfläche (200) umfasst, wobei die Daten Patientendaten enthalten,
- Zugriff auf eine erste Datenbank (122), wobei die erste Datenbank (122) medizinische Informationsobjekte enthält, wobei die medizinischen Informationsobjekte mit Abfragebedingungen bezüglich der empfangenen Daten verknüpft sind, wobei die erste Datenbank (122) in dem Datenverarbeitungssystem enthalten ist,
- Überprüfung ob zumindest eine der Abfragebedingungen erfüllt ist,
- Erzeugung eines Popups (134) auf der graphischen Benutzeroberfläche (200), wenn zumindest eine der Abfragebedingungen erfüllt ist, wobei das Popup (134) die Informationsobjekte aufweist, für welche die Abfragebedingung erfüllt sind, wobei ein Fensterfokus auf dem ersten Anzeigefenster erhalten bleibt und wobei das Popup (134) auf der graphischen Benutzeroberfläche (200) so angezeigt wird, dass eine weitere Eingabe von Daten in dem ersten Anzeigefenster ermöglicht wird.

2. Verfahren nach Anspruch 1, wobei das Popup (134) ferner eine Verknüpfung mit weiterführenden Informationen aufweist, wobei bei einer Benutzeraktivierung der Verknüpfung ein Lesen der weiterführenden Informationen von einer Informationsquelle erfolgt und eine Ausgabe der ausgelesenen weiterführenden Information in einem zweiten Anzeigefenster (300) auf der graphischen Benutzeroberfläche (200) erfolgt.

3. Verfahren nach Anspruch 2, wobei es sich bei der Informationsquelle um die erste Datenbank (122) oder eine dem Datenverarbeitungssystem externe Datenquelle (128; 126; 124) handelt.

4. Verfahren nach Anspruch 3, wobei es sich bei der externen Datenquelle um das Internet (128) und/oder um eine zweite Datenbank (126) handelt.

5. Verfahren nach einem der vorigen Ansprüche, wobei das Popup (134) von der graphischen Benutzeroberfläche (200) nach einem vorgegebenen ersten Zeitintervall entfernt wird.

6. Verfahren nach einem der vorigen Ansprüche, wobei die weiterführenden Informationen in der Datenbank als HTML oder XML Dokumente gespeichert sind.

7. Verfahren nach einem der vorigen Ansprüche, wobei es sich bei dem zweiten Anzeigefenster (300) um ein Fenster eines Webbrowsers handelt.

8. Verfahren nach einem der vorigen Ansprüche, wobei die von dem ersten Anzeigefenster empfangenen Daten Informationen über den Benutzer der grafischen Benutzeroberfläche enthalten.

9. Verfahren nach Anspruch 8, wobei die Informationen über den Benutzer Praxisstammdaten und/oder eine Facharztgruppe und/oder eine Benutzerkennung umfassen.

10. Verfahren nach einem der vorigen Ansprüche, ferner mit dem Schritt des Abfragens ergänzender zu dem Patienten zugehöriger Patientendaten von einer dritten Datenbank (124; 126; 128) , wobei die dritte Datenbank Teil des Datenverarbeitungssystems (100) ist oder eine externe Datenbank ist, wobei die medizinischen Informationsobjekte zusätzlich mit Abfragebedingungen bezüglich der abgefragten ergänzenden Patientendaten verknüpft sind.

11. Verfahren nach einem der vorigen Ansprüche, wobei die Abfragebedingungen personalisierte Benutzerabfragebedingungen und/oder personalisierte Patientenabfragebedingungen umfassen, wobei die Benutzerabfragebedingungen und Patientenabfragebedingungen dynamisch in der ersten Datenbank ergänzt werden.

12. Verfahren nach Anspruch 11, wobei das Popup (134) ferner zumindest eine durch den Benutzer auswählbare Anzeigeoption aufweist, wobei bei einer Auswahl der Anzeigeoption die personalisierte Benutzerabfragebedingung und/oder Patientenabfragebedingung erzeugt wird, wobei in die personalisierte Benutzerabfragebedingung und/oder Patientenabfragebedingung die durch den Benutzer ausgewählte Anzeigeoption eingeht.

13. Verfahren nach Anspruch 12, wobei es sich bei der Anzeigeoption
- um das sofortige Ausblenden und Wiedereinblenden des Popups (134) nach einem vorgegebenen oder auswählbaren zweiten Zeitintervall handelt und/oder
- um das sofortige Ausblenden des Popups (134) und nicht-Wiedereinblenden handelt.

14. Verfahren nach einem der vorigen Ansprüche, wobei die empfangenen Daten Datenobjekte aufweisen, wobei
- beim Empfang der Daten die Datenobjekte in einem ersten Stack (408) gespeichert werden,
- aus einer ersten Tabelle (400) für die in dem ersten Stack (408) gespeicherten Datenobjekte diesen Datenobjekten in der ersten Tabelle (400) zugeordnete Kennungen ausgelesen werden, wobei jeder Kennung eines der medizinischen Informationsobjekte zugewiesen ist,
- in einem zweiten Stack (406) zu jeder Kennung die Anzahl der in dem ersten Stack gespeicherten Datenobjekte gespeichert wird, welche in der ersten Tabelle dieser Kennung zugeordnet sind,
- die Abfragebedingung erfüllt ist, wenn die zu einer Kennung in dem zweiten Stack gespeicherte Anzahl an empfangenen Datenobjekten einer vorgegebenen Anzahl an Datenobjekten entspricht, welche in einer zweiten Tabelle (402) zu der Kennung zugeordnet sind.

15. Verfahren nach Anspruch 14, wobei die Speicherung eines empfangenen Datenobjekts in dem ersten Stack (408) nur dann erfolgt, wenn das Datenobjekt noch nicht in dem ersten Stack gespeichert ist.

16. Datenverarbeitungssystem zur kontextsensitiven Bereitstellung von patientenbezogenen Informationen auf einer graphischen Benutzeroberfläche (200) des Datenverarbeitungssystems (100), wobei das Datenverarbeitungssystem umfasst::
- Mittel (114) zum Empfang von Daten von einem Anwendungsprogramm (108), wobei das Anwendungsprogramm (108) ein erstes Anzeigefenster (132) auf der graphischen Benutzeroberfläche (200) umfasst, wobei die Daten Patientendaten enthalten,
- Mittel zum Zugriff auf eine erste Datenbank (122), wobei die erste Datenbank (122) medizinische Informationsobjekte enthält, wobei die medizinischen Informationsobjekte mit Abfragebedingungen bezüglich der empfangenen Daten verknüpft sind, wobei die erste Datenbank (122) in dem Datenverarbeitungssystem enthalten ist,
- Mittel (118) zur Überprüfung ob zumindest eine der Abfragebedingungen erfüllt ist,
- Mittel (120) zur Erzeugung eines Popups (134) auf der graphischen Benutzeroberfläche (200), wenn zumindest eine der Abfragebedingungen erfüllt ist, wobei das Popup (134) die Informationsobjekte aufweist, für welche die Abfragebedingung erfüllt sind, wobei die Mittel zur Erzeugung des Popups (134) so ausgebildet sind, dass ein Fensterfokus auf dem ersten Anzeigefenster erhalten bleibt, und dass das Popup (134) auf der graphischen Benutzeroberfläche (200) so angezeigt wird, dass eine weitere Eingabe von Daten in dem ersten Anzeigefenster nicht beeinträchtigt wird.

17. Datenverarbeitungssystem nach Anspruch 16, ferner mit Mitteln zum Abfragen ergänzender zu dem Patienten zugehöriger Patientendaten von einer dritten Datenbank, wobei die dritte Datenbank Teil des Datenverarbeitungssystems (100) ist oder eine externe Datenbank ist, wobei die medizinischen Informationsobjekte zusätzlich mit Abfragebedingungen bezüglich der abgefragten ergänzenden Patientendaten verknüpft sind.

18. Datenverarbeitungssystem nach einem der vorigen Ansprüche 16 oder 17, wobei die empfangenen Daten Datenobjekte aufweisen, ferner mit
- Mitteln zum Speichern der Datenobjekte beim Empfang der Daten in einem ersten Stack (408),
- Mittel zum Auslesen von Kennungen aus einer ersten Tabelle (400), wobei in der ersten Tabelle die Kennungen den in dem ersten Stack (408) gespeicherten Datenobjekten zugeordnet sind, wobei jeder Kennung eines der medizinischen Informationsobjekte zugewiesen ist,
- Mittel zur Speicherung zu jeder Kennung die Anzahl der in dem ersten Stack gespeicherten Datenobjekte in einem zweiten Stack, wobei die Abfragebedingung erfüllt ist, wenn die zu einer Kennung in dem zweiten Stack (406) gespeicherte Anzahl an empfangenen Datenobjekten einer vorgegebenen Anzahl an Datenobjekten entspricht, welche in einer zweiten Tabelle (402) zu der Kennung zugeordnet sind.

19. Computerprogrammprodukt mit von einem Prozessor ausführbaren Instruktionen zur Durchführung des Verfahrens nach einem der Ansprüche 1 bis 15.

## Geänderte Patentansprüche

### Geänderte Patentansprüche gemäss Regel 137(2) EPÜ.

**1.** Verfahren zur kontextsensitiven Bereitstellung von patientenbezogenen Informationen auf einer graphischen Benutzeroberfläche eines Datenverarbeitungssystems (100) mit den folgenden Schritten:
- Empfang von Daten von einem Anwendungsprogramm (108), wobei das Anwendungsprogramm (108) ein erstes Anzeigefenster (132) auf der graphischen Benutzeroberfläche (200) umfasst, wobei die Daten Patientendaten enthalten,
- Zugriff auf eine erste Datenbank (122), wobei die erste Datenbank (122) medizinische Informationsobjekte enthält, wobei es sich bei den medizinischen Informationsobjekten um medizinische Informationen handelt, wobei die erste Datenbank (122) auf die empfangen Daten anwendbare Abfragebedingungen enthält, wobei die medizinischen Informationsobjekte den Abfragebedingungen zugeordnet sind, wobei es sich bei den Abfragebedingungen um Abfragen handelt, wobei die erste Datenbank (122) in dem Datenverarbeitungssystem enthalten ist,
- Überprüfung ob zumindest eine der Abfragebedingungen erfüllt ist,
- Erzeugung eines Popups (134) auf der graphischen Benutzeroberfläche (200), wenn zumindest eine der Abfragebedingungen erfüllt ist, wobei das Popup (134) die Informationsobjekte aufweist, für welche die Abfragebedingung erfüllt sind, wobei ein Fensterfokus auf dem ersten Anzeigefenster erhalten bleibt und wobei das Popup (134) auf der graphischen Benutzeroberfläche (200) so angezeigt wird, dass eine weitere Eingabe von Daten in dem ersten Anzeigefenster ermöglicht wird,
wobei die Abfragebedingungen personalisierte Benutzerabfragebedingungen und/oder personalisierte Patientenabfragebedingungen umfassen, wobei die Benutzerabfragebedingungen und Patientenabfragebedingungen dynamisch in der ersten Datenbank (122) ergänzt werden, wobei das Popup (134) ferner zumindest eine durch den Benutzer auswählbare Anzeigeoption aufweist,
wobei bei einer Auswahl der Anzeigeoption die personalisierte Benutzerabfragebedingung und/oder Patientenabfragebedingung erzeugt wird, wobei in die personalisierte Benutzerabfragebedingung und/oder Patientenabfragebedingung die durch den Benutzer ausgewählte Anzeigeoption eingeht.

**2.** Verfahren nach Anspruch 1, wobei das Popup (134) ferner eine Verknüpfung mit weiterführenden Informationen aufweist, wobei bei einer Benutzeraktivierung der Verknüpfung ein Lesen der weiterführenden Informationen von einer Informationsquelle erfolgt und eine Ausgabe der ausgelesenen weiterführenden Information in einem zweiten Anzeigefenster (300) auf der graphischen Benutzeroberfläche (200) erfolgt.

**3.** Verfahren nach Anspruch 2, wobei es sich bei der Informationsquelle um die erste Datenbank (122) oder eine dem Datenverarbeitungssystem externe Datenquelle (128; 126; 124) handelt.

**4.** Verfahren nach Anspruch 3, wobei es sich bei der externen Datenquelle um das Internet (128) und/oder um eine zweite Datenbank (126) handelt.

**5.** Verfahren nach einem der vorigen Ansprüche, wobei das Popup (134) von der graphischen Benutzeroberfläche (200) nach einem vorgegebenen ersten Zeitintervall entfernt wird.

**6.** Verfahren nach einem der vorigen Ansprüche, wobei die weiterführenden Informationen in der Datenbank als HTML oder XML Dokumente gespeichert sind.

**7.** Verfahren nach einem der vorigen Ansprüche, wobei es sich bei dem zweiten Anzeigefenster (300) um ein Fenster eines Webbrowsers handelt.

**8.** Verfahren nach einem der vorigen Ansprüche, wobei die von dem ersten Anzeigefenster empfangenen Daten Informationen über den Benutzer der grafischen Benutzeroberfläche enthalten.

**9.** Verfahren nach Anspruch 8, wobei die Informationen über den Benutzer Praxisstammdaten und/oder eine Facharztgruppe und/oder eine Benutzerkennung umfassen.

**10.** Verfahren nach einem der vorigen Ansprüche, ferner mit dem Schritt des Abfragens ergänzender zu dem Patienten zugehöriger Patientendaten von einer dritten Datenbank (124; 126; 128), wobei die dritte Datenbank Teil des Datenverarbeitungssystems (100) ist oder eine externe Datenbank ist, wobei den medizinischen Informationsobjekten zusätzlich Abfragebedingungen bezüglich der abgefragten ergänzenden Patientendaten zugeordnet sind.

**11.** Verfahren nach Anspruch 1, wobei es sich bei der Anzeigeoption
- um das sofortige Ausblenden und Wiedereinblenden des Popups (134) nach einem vorgegebenen oder auswählbaren zweiten Zeitintervall handelt und/oder
- um das sofortige Ausblenden des Popups (134) und nicht-Wiedereinblenden handelt.

**12.** Verfahren nach einem der vorigen Ansprüche, wobei die empfangenen Daten Datenobjekte aufweisen, wobei
- beim Empfang der Daten die Datenobjekte in einem ersten Stack (408) gespeichert werden,
- aus einer ersten Tabelle (400) für die in dem ersten Stack (408) gespeicherten Datenobjekte diesen Datenobjekten in der ersten Tabelle (400) zugeordnete Kennungen ausgelesen werden, wobei jeder Kennung eines der medizinischen Informationsobjekte zugewiesen ist,
- in einem zweiten Stack (406) zu jeder Kennung die Anzahl der in dem ersten Stack gespeicherten Datenobjekte gespeichert wird, welche in der ersten Tabelle dieser Kennung zugeordnet sind,
- die Abfragebedingung erfüllt ist, wenn die zu einer Kennung in dem zweiten Stack gespeicherte Anzahl an empfangenen Datenobjekten einer vorgegebenen Anzahl an Datenobjekten entspricht, welche in einer zweiten Tabelle (402) zu der Kennung zugeordnet sind.

**13.** Verfahren nach Anspruch 12, wobei die Speicherung eines empfangenen Datenobjekts in dem ersten Stack (408) nur dann erfolgt, wenn das Datenobjekt noch nicht in dem ersten Stack gespeichert ist.

**14.** Datenverarbeitungssystem zur kontextsensitiven Bereitstellung von patientenbezogenen Informationen auf einer graphischen Benutzeroberfläche (200) des Datenverarbeitungssystems (100), wobei das Datenverarbeitungssystem umfasst::
- Mittel (114) zum Empfang von Daten von einem Anwendungsprogramm (108), wobei das Anwendungsprogramm (108) ein erstes Anzeigefenster (132) auf der graphischen Benutzeroberfläche (200) umfasst, wobei die Daten Patientendaten enthalten,
- Mittel zum Zugriff auf eine erste Datenbank (122), wobei die erste Datenbank (122) medizinische Informationsobjekte enthält, wobei es sich bei den medizinischen Informationsobjekten um medizinische Informationen handelt, wobei die erste Datenbank (122) auf die empfangen Daten anwendbare Abfragebedingungen enthält, wobei die medizinischen Informationsobjekte den Abfragebedingungen zugeordnet sind, wobei es sich bei den Abfragebedingungen um Abfragen handelt, wobei die erste Datenbank (122) in dem Datenverarbeitungssystem enthalten ist,
- Mittel (118) zur Überprüfung ob zumindest eine der Abfragebedingungen erfüllt ist,
- Mittel (120) zur Erzeugung eines Popups (134) auf der graphischen Benutzeroberfläche (200), wenn zumindest eine der Abfragebedingungen erfüllt ist, wobei das Popup (134) die Informationsobjekte aufweist, für welche die Abfragebedingung erfüllt sind, wobei die Mittel zur Erzeugung des Popups (134) so ausgebildet sind, dass ein Fensterfokus auf dem ersten Anzeigefenster erhalten bleibt, und dass das Popup (134) auf der graphischen Benutzeroberfläche (200) so angezeigt wird, dass eine weitere Eingabe von Daten in dem ersten Anzeigefenster nicht beeinträchtigt wird,
wobei die Abfragebedingungen personalisierte Benutzerabfragebedingungen und/oder personalisierte Patientenabfragebedingungen umfassen, wobei die Benutzerabfragebedingungen und Patientenabfragebedingungen dynamisch in der ersten Datenbank ergänzt werden, wobei das Popup (134) ferner zumindest eine durch den Benutzer auswählbare Anzeigeoption aufweist,
wobei bei einer Auswahl der Anzeigeoption die personalisierte Benutzerabfragebedingung und/oder Patientenabfragebedingung erzeugt wird, wobei in die personalisierte Benutzerabfragebedingung und/oder Patientenabfragebedingung die durch den Benutzer ausgewählte Anzeigeoption eingeht.

**15.** Datenverarbeitungssystem nach Anspruch 14, ferner mit Mitteln zum Abfragen ergänzender zu dem Patienten zugehöriger Patientendaten von einer dritten Datenbank, wobei die dritte Datenbank Teil des Datenverarbeitungssystems (100) ist oder eine externe Datenbank ist, wobei die medizinischen Informationsobjekte zusätzlich mit Abfragebedingungen bezüglich der abgefragten ergänzenden Patientendaten verknüpft sind.

**16.** Datenverarbeitungssystem nach einem der vorigen Ansprüche 14 oder 15,
wobei die empfangenen Daten Datenobjekte aufweisen, ferner mit
- Mitteln zum Speichern der Datenobjekte beim Empfang der Daten in einem ersten Stack (408),
- Mittel zum Auslesen von Kennungen aus einer ersten Tabelle (400), wobei in der ersten Tabelle die Kennungen den in dem ersten Stack (408) gespeicherten Datenobjekten zugeordnet sind, wobei jeder Kennung eines der medizinischen Informationsobjekte zugewiesen ist,
- Mittel zur Speicherung zu jeder Kennung die Anzahl der in dem ersten Stack gespeicherten Datenobjekte in einem zweiten Stack, wobei die Abfragebedingung erfüllt ist, wenn die zu einer Kennung in dem zweiten Stack (406) gespeicherte Anzahl an empfangenen Datenobjekten einer vorgegebenen Anzahl an Datenobjekten entspricht, welche in einer zweiten Tabelle (402) zu der Kennung zugeordnet sind.

**17.** Computerprogrammprodukt mit von einem Prozessor ausführbaren Instruktionen zur Durchführung des Verfahrens nach einem der Ansprüche 1 bis 13.
